(19) Europäisches Patentamt
European Patent Office
Office européen des brevets

(11) **EP 4 197 445 B1**

(12) **EUROPEAN PATENT SPECIFICATION**

(45) Date of publication and mention
of the grant of the patent:
**03.04.2024 Bulletin 2024/14**

(21) Application number: **22207427.0**

(22) Date of filing: **15.11.2022**

(51) International Patent Classification (IPC):
***A61B 6/00*** *(2024.01)*

(52) Cooperative Patent Classification (CPC):
**A61B 6/4035; A61B 6/4291; A61B 6/484;
A61B 6/5241**

(54) **RADIOGRAPHIC IMAGING APPARATUS, IMAGE GENERATION METHOD, RADIOGRAPHIC IMAGING SYSTEM, AND PROGRAM**

RÖNTGENBILDGEBUNGSVORRICHTUNG, BILDERZEUGUNGSVERFAHREN, RÖNTGENBILDGEBUNGSSYSTEM UND PROGRAMM

APPAREIL D'IMAGERIE RADIOGRAPHIQUE, PROCÉDÉ DE GÉNÉRATION D'IMAGE, SYSTÈME D'IMAGERIE RADIOGRAPHIQUE ET PROGRAMME

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.12.2021 JP 2021203609**

(43) Date of publication of application:
**21.06.2023 Bulletin 2023/25**

(73) Proprietor: **KONICA MINOLTA, INC.**
**Tokyo 100-7015 (JP)**

(72) Inventor: **KIKUCHI, Ryouhei**
**Tokyo, 100-7015 (JP)**

(74) Representative: **Gille Hrabal**
**Partnerschaftsgesellschaft mbB**
**Patentanwälte**
**Brucknerstraße 20**
**40593 Düsseldorf (DE)**

(56) References cited:
**US-A1- 2016 042 533     US-A1- 2020 333 265
US-A1- 2021 015 437**

**Description**

BACKGROUND

TECHNOLOGICAL FIELD

The present invention relates to a radiographic imaging apparatus, an image generation method, a radiographic imaging system, and a program.

DESCRIPTION OF THE RELATED ART

In related art, an X-ray imaging apparatus using a Talbot interferometer or a Talbot-Lau interferometer having a plurality of gratings (hereinafter, referred to as an X-ray Talbot imaging apparatus) is known as an X-ray imaging apparatus. In the X-ray Talbot imaging apparatus, an imaging range is determined by a size (area) of a grating. However, it is difficult to manufacture a grating, and it is extremely difficult to increase a size of the grating. Thus, in a case where a subject of a size greater than an imaging range of one time is imaged, it is necessary to perform imaging while shifting a position of the subject, then align positions of images and combine the images through image processing.

Further, oriented imaging is also performed in which a plurality of images having different directions with respect to the gratings are captured by rotating a subject and information such as an orientation degree that cannot be known only from each image can be derived by performing image processing. Further, the positions are aligned by correcting displacement occurring when the subject is rotated in the image processing.

Regarding position alignment, the invention disclosed in JP 6780591 B discloses aligning positions using an absorption image. Further, typically, positions are sometimes aligned using a marker created with a substance (such as lead) that absorbs a large X-ray amount. Another example of known radiographic imaging devices is shown in US2016/042533 A1, which shows a radiographic imaging apparatus with a grating, where images are reconstructed based on Moire fringe images.

SUMMARY

However, in a case where the subject has a size not falling within an imaging range of one time and is a flat plate, or the like, not having significant characteristics, it is difficult to align positions with high accuracy. While positions are aligned through match of pixels of the absorption image in the invention disclosed in JP 6780591 B, in a case of a flat plate not having particularly significant characteristics, there is a case where images may be displaced by several pixels. Further, in a case where a marker is used, there is a problem that the marker comes out at a position of the marker on the captured image, which may cause a defect.

It is therefore an object of the present invention to provide a radiographic imaging apparatus capable of aligning positions of images captured a plurality of times with high accuracy even for a subject not having significant characteristics on the images and having a size exceeding an imaging range of one time, an image generation method, a radiographic imaging system, and a program.

To achieve at least one of the abovementioned objects, according to an aspect of the present invention, a radiographic imaging apparatus reflecting one aspect of the present invention is a radiographic imaging apparatus in which a radiation source, a plurality of gratings, and a radiation detector are provided side by side in a radiation irradiation axis direction, the radiographic imaging apparatus including:

  a generator that generates a reconstructed image on the basis of a Moire fringe image obtained by performing imaging by irradiating a subject disposed at a position overlapping the radiation irradiation axis direction with radiation by the radiation source;
  an acquirer that acquires relative position information that is relative positions in two or more dimensions of the subject with respect to the gratings; and
  a controller that associates the reconstructed image with the relative position information.

To achieve at least one of the abovementioned objects, according to an aspect of the present invention, an image generation method reflecting one aspect of the present invention is an image generation method to be performed by a radiographic imaging apparatus in which a radiation source, a plurality of gratings, and a radiation detector are provided side by side in a radiation irradiation axis direction, the image generation method including:

  generating, by a generator, a reconstructed image on the basis of a Moire fringe image obtained by performing imaging by irradiating a subject disposed at a position overlapping the radiation irradiation axis direction with radiation by the radiation source;
acquiring, by an acquirer, relative position information that is relative positions in two or more dimensions of the subject with respect to the gratings; and
associating, by a controller, the reconstructed image with the relative position information.

Preferably, a radiographic imaging system of the present invention may be a radiographic imaging system including:

the radiographic imaging apparatus, and
an apparatus that outputs image data from which an imaging range and the subject can be grasped in the same coordinate system,
wherein positions of an analysis result of the apparatus are made to match positions of the reconstructed image using the relative position information.

Preferably, a radiographic imaging system of the present invention may be a radiographic imaging system including the radiographic imaging apparatus,
wherein positions of the reconstructed image and an optical image obtained by the imager are aligned using the relative position information, and the reconstructed image and the optical image are displayed in a superimposed manner.

To achieve at least one of the abovementioned objects, according to an aspect of the present invention, a program reflecting one aspect of the present invention causes a computer of a radiographic imaging apparatus in which a radiation source, a plurality of gratings and a radiation detector are provided side by side in a radiation irradiation axis direction, to function as:

a generator that generates a reconstructed image on the basis of a Moire fringe image obtained by performing imaging by irradiating a subject disposed at a position overlapping the radiation irradiation axis direction with radiation by the radiation source;
an acquirer that acquires relative position information that is relative positions in two or more dimensions of the subject with respect to the gratings; and
a controller that associates the reconstructed image with the relative position information.

BRIEF DESCRIPTION OF THE DRAWINGS

The advantages and features provided by one or more embodiments of the invention will become more fully understood from the detailed description given hereinbelow and the appended drawings which are given by way of illustration only, and thus are not intended as a definition of the limits of the present invention, wherein:

FIG. 1 is a view illustrating a configuration example of an X-ray Talbot imaging apparatus according to a first embodiment;
FIG. 2 is a plan view of a multi-slit;
FIG. 3 is a block diagram illustrating a functional configuration of a controller;
FIG. 4 is a view for explaining principle of a Talbot interferometer;
FIG. 5 is a flowchart illustrating imaging processing a to be executed in the first embodiment (Example 1);
FIG. 6 is a view illustrating a relationship between a subject and an imaging position in the first embodiment (Example 1);
FIG. 7 is a view illustrating a relationship between a subject and an imaging position in Example 2;
FIG. 8 is a view illustrating a relationship between a subject and an imaging position in Example 3;
FIG. 9 is a view illustrating a relationship between a parent sample and child samples in Example 4;
FIG. 10 is a flowchart illustrating imaging possible/impossible determination processing b to be executed in Example 5;
FIG. 11 is an example of derivation of a position correction amount;
FIG. 12A is a view illustrating a configuration example of an X-ray Talbot imaging apparatus according to a second embodiment;
FIG. 12B is another view illustrating the configuration example of the X-ray Talbot imaging apparatus according to the second embodiment;
FIG. 13 is Display example 1 in the second embodiment;
FIG. 14 is Display example 2 in the second embodiment; and
FIG. 15 is a view illustrating a configuration example of an X-ray Talbot imaging apparatus according to a third embodiment.

## DETAILED DESCRIPTION OF THE EMBODIMENTS

Embodiments of the present invention will be described below with reference to the drawings. However, the scope of the invention is not limited to the disclosed embodiments.

[First embodiment]

(Configuration of radiographic imaging system)

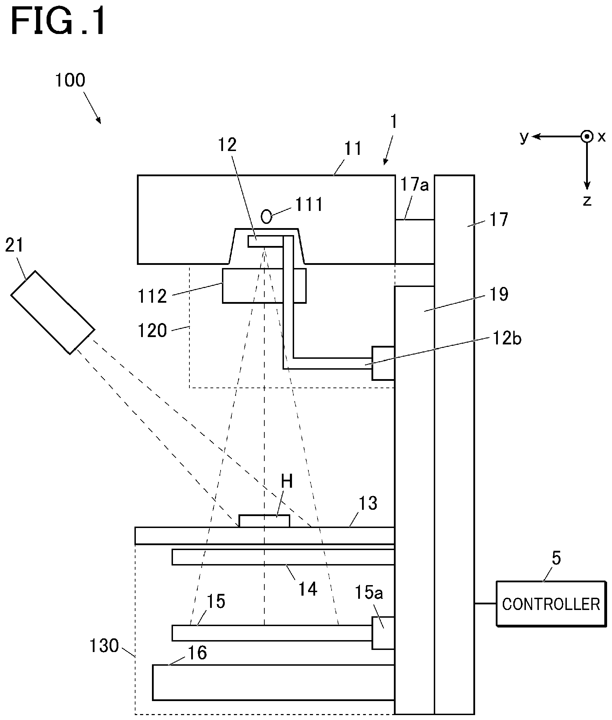

FIG. 1 is a view schematically illustrating an X-ray Talbot imaging apparatus 100 according to a first embodiment of the present invention.

As illustrated in FIG. 1, the X-ray Talbot imaging apparatus 100 includes a body 1 and a controller 5.

As illustrated in FIG. 1, the body 1 includes a Talbot-Lau interferometer including a radiation source 11, a first cover unit 120 including a multi-slit 12 and an added filter/collimator 112, a second cover unit 130 including a subject stand 13, a first grating 14, a second grating 15 and a radiation detector 16, a supporting column 17, and a base 19. The Talbot-Lau interferometer of the body 1 is a vertical interferometer, and the radiation source 11, the multi-slit 12, the subject stand 13, the first grating 14, the second grating 15 and the radiation detector 16 are disposed in this order in a z direction that is a (vertical) direction of gravitational force. Further, a camera 21 is provided at a location deviating from an X-ray irradiation range.

The multi-slit 12, the subject stand 13, the first grating 14, the second grating 15 and the radiation detector 16 are held on the same base 19 and attached to the supporting column 17. The base 19 may be constituted so as to movable in the z direction with respect to the supporting column 17.

Further, in addition to the base 19, the radiation source 11 is attached to the supporting column 17. The radiation source 11 is held at the supporting column 17 via a buffer material 17a. The buffer material 17a may be any material if the material can absorb impact and vibration, and may be, for example, elastomer, or the like. The radiation source 11 produces heat by irradiation of radiation, and thus, the buffer material 17a on the radiation source 11 side is further preferably a heat insulating material.

The radiation source 11 includes an X-ray tube and generates an X-ray by the X-ray tube and radiates the X-ray in the z direction (direction of gravitational force). As the X-ray tube, for example, a Coolidge X-ray tube or a rotating anode X-ray tube can be used. As an anode, tungsten or molybdenum can be used.

A focal diameter of the radiation source 11 is preferably 0.03 to 3 (mm), and more preferably 0.1 to 1 (mm).

Note that while in the present embodiment, a case where imaging is performed using an X-ray will be described as an example, other kinds of radiation, for example, a neutron ray, a gamma ray, or the like, may be used.

The first cover unit 120 is a unit provided immediately below the radiation source 11. As illustrated in FIG. 1, the first cover unit 120 includes the multi-slit 12, an attachment arm 12b, the added filter/collimator 112, and the like. The respective components of the first cover unit 120 are protected by being covered with a cover material.

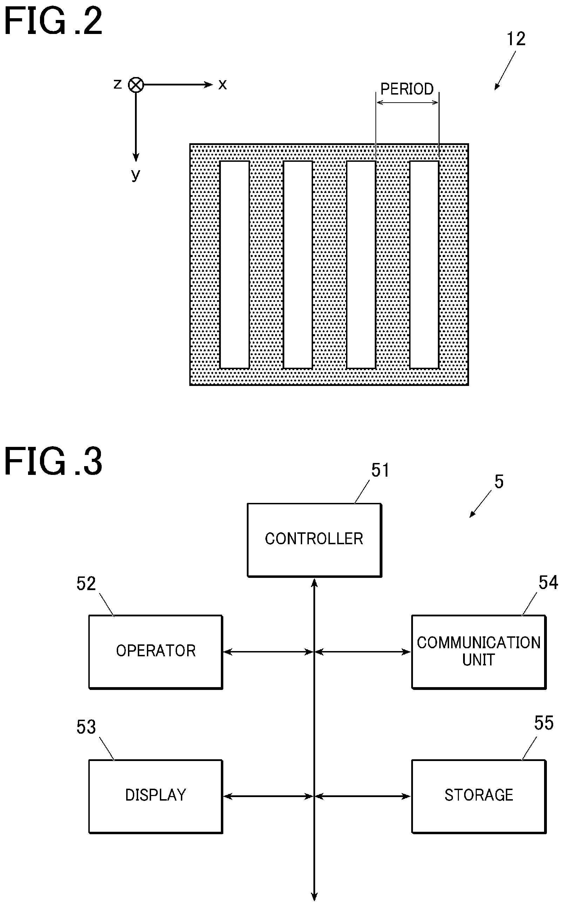

The multi-slit 12 (G0 grating) is a diffraction grating, and as illustrated in FIG. 2, a plurality of slits are arranged and provided at regular intervals in an x direction orthogonal to a radiation irradiation axis direction (here, the z direction). The multi-slit 12 is formed on a substrate having a low radiation absorption rate such as silicon and glass, with a high-radiation shielding material, that is, a material having a high radiation absorption rate such as tungsten, lead and gold. For example, a resist layer is masked in a slit shape through photolithography, and UV is radiated so that a pattern of the slits is transferred to the resist layer. Slit structures having the same shape as the pattern are obtained through exposure, and a metal is embedded between the slit structures by electroforming, thereby the multi-slit 12 is formed.

A slit period (grating period) of the multi-slit 12 is 1 to 60 ($\mu$m). The slit period is set so that a distance between adjacent slits becomes one period as illustrated in FIG. 2. A width of the slit (a length of each slit in a slit period direction (x direction)) is a length of 1 to 60 (%) of the slit period, and more preferably 10 to 40 (%). A height of the slit (a height in the z direction) is 1 to 1500 ($\mu$m) and preferably 30 to 1000 ($\mu$m). The multi-slit 12 is supported by the attachment arm 12b and attached to the base 19. Note that the multi-slit 12 (G0 grating) does not have to be provided if a focus of the X-ray tube of the radiation source 11 is small, for example, like a micro focus ray source.

The added filter/collimator 112 limits an irradiation region of the X-ray to be radiated from the radiation source 11 and removes a low energy component that does not contribute to imaging among the X-ray radiated from the radiation source 11.

As illustrated in FIG. 1, the second cover unit 130 includes the subject stand 13, the first grating 14, the second grating 15, a movement mechanism 15a, the radiation detector 16, and the like. The second cover unit 130, which has an upper surface that serves as the subject stand 13, protects internal components from damage by contact of a subject H or an engineer and intrusion of dust by covering the periphery of the subject stand 13 with a cover member. Further, a temperature inside the unit is less likely to be affected by the influence of ambient air, so that it is possible to reduce fluctuation of grating positions due to thermal expansion, or the like, of the first grating 14 and the second grating 15.

The subject stand 13 is a stand on which the subject H is to be placed. Further, the subject stand 13 is movable in a two-dimensional direction + rotation (XY axis + Θ axis).

The first grating 14 (G1 grating) is a diffraction grating in which a plurality of slits are arranged and provided in the x direction orthogonal to the z direction that is the radiation irradiation axis direction in a similar manner to the multi-slit 12. The first grating 14 can be formed through photolithography using UV in a similar manner to the multi-slit 12, or a grating structure may be formed only with silicon by performing deep drilling processing on a silicon substrate with a fine wire using a so-called ICP method. A slit period of the first grating 14 is 1 to 20 ($\mu$m). A width of the slit is 20 to 70 (%) of the slit period and preferably 35 to 60 (%). A height of the slit is 1 to 100 ($\mu$m).

The second grating 15 (G2 grating) is a diffraction grating in which a plurality of slits are arranged and provided in the x direction orthogonal to the z direction that is the radiation irradiation axis direction in a similar manner to the multi-slit 12. The second grating 15 can also be formed through photolithography. A slit period of the second grating 15 is 1 to 20 ($\mu$m). A width of the slit is 30 to 70 (%) of the slit period and preferably 35 to 60 (%). A height of the slit is 1 to 100 ($\mu$m). The movement mechanism 15a that moves the second grating 15 in the x direction is provided adjacent to the second grating 15. As the movement mechanism 15a, a mechanism having any configuration may be used if the mechanism can linearly move the second grating 15 in the x direction through driving by a motor, or the like.

The radiation detector 16 in which conversion elements that generate electrical signals in accordance with radiated radiation are disposed in two dimensions, reads the electrical signals generated by the conversion elements as image signals. A pixel size of the radiation detector 16 is 10 to 300 ($\mu$m) and more preferably 50 to 200 ($\mu$m). The radiation detector 16 is preferably fixed at the base 19 so as to abut on the second grating 15. This is because as a distance between the second grating 15 and the radiation detector 16 becomes greater, a Moire fringe image obtained by the radiation detector 16 becomes more blurred.

As the radiation detector 16, a flat panel detector (FPD) can be used. While the FPD includes an indirect conversion type in which radiation is converted into an electrical signal using a photoelectric conversion element via a scintillator, and a direct conversion type in which radiation is directly converted into an electrical signal, either may be used.

Further, as the radiation detector 16, a radiation detector in which an intensity modulation effect of the second grating 15 is provided may be used. For example, in order to provide a dead region with a period and a width equivalent to the period and the width of the slit of the second grating 15 to the scintillator, a slit scintillator detector obtained by drilling a groove in the scintillator to obtain a grid-like scintillator may be used as the radiation detector 16. In this case, the radiation detector 16 serves as both the second grating 15 and the radiation detector 16, and thus, it is not necessary to separately provide the second grating 15. In other words, the X-ray Talbot imaging apparatus including the slit scintillator detector is equivalent to the X-ray Talbot imaging apparatus including the second grating 15 and the radiation detector 16.

Note that while a case has been described where the Talbot-Lau interferometer of the body 1 is configured so that the subject H on a lower side is irradiated with an X-ray from the radiation source 11 provided on an upper side (a case of a vertical type), the present invention is not limited to this, and the Talbot-Lau interferometer may be configured so that the subject H on an upper side is irradiated with an X-ray from the radiation source 11 provided on a lower side. Further, the Talbot-Lau interferometer may be configured so that an X-ray is radiated in an arbitrary direction such as a horizontal direction (a case of a so-called horizontal type).

The camera 21, which can acquire images including depth information, is an RGB-D camera (red green blue-depth camera) that can obtain an RGB-D image including three-dimensional point cloud data. In other words, the camera 21 can acquire three-dimensional information and the RGB image (two-dimensional image).

Further, the camera 21 is adjusted in accordance with an absolute position of the X-ray Talbot imaging apparatus 100 (G0 grating, G1 grating, G2 grating). Specifically, coordinates of the RGB-D image captured by the camera 21 are made to match coordinates of the reconstructed image captured and generated by the radiation detector 16. For example, X-ray high-absorbent markers are disposed at five locations of four corners and the center of the imaging range on the subject stand 13, and coordinates of the captured RGB-D image are made to match coordinates of the absorption image.

Thus, the camera 21 functions as an imager that captures an image from which relative positions (relative position information) of two or more dimensions of the subject with respect to the gratings can be acquired.

Note that the relative position information may be acquired by utilizing a plurality of cameras. For example, there is a case where means for acquiring a two-dimensional image of the subject H and means for acquiring a three-dimensional image are individually provided. Further, the relative position information may be acquired from movement information of a robot arm by putting the subject H into the robot arm. Further, the relative position information may be acquired with high accuracy using a 3D scanner. Still further, if the subject H is a flat plate, thickness information may be obtained by performing imaging from the side with a camera (combination of a normal camera from above and a normal camera from the side).

As illustrated in FIG. 3, the controller 5 includes a controller 51, an operator 52, a display 53, a communication unit 54 and a storage 55.

The controller 51 includes a central processing unit (CPU), a random access memory (RAM), and the like. The controller 51 is connected to the respective components of the body 1 (for example, the radiation source 11, the radiation detector 16, the movement mechanism 15a and the camera 21) and controls operation of the respective components. Further, the controller 51 executes various kinds of processing including imaging processing which will be described later in cooperation with a program stored in the storage 55.

Further, the controller 51 functions as a generator that generates a reconstructed image on the basis of a Moire fringe image obtained by performing imaging by irradiating the subject H disposed at a position overlapping the radiation irradiation axis direction with radiation by the radiation source 11 in cooperation with the radiation source 11, the gratings (the multi-slit 12, the first grating 14, the second grating 15), the radiation detector 16, and the like. Further, the controller 51 functions as an acquirer that acquires relative position information that is relative positions in two or more dimensions of the subject H with respect to the gratings. Still further, the controller 51 functions as a controller that associates the reconstructed image which will be described later with the relative position information.

The operator 52, which includes a touch panel integrally constituted with a display of the display 53 in addition to an exposure switch and keys to be used for input operation such as imaging conditions, generates an operation signal in accordance with the operation and outputs the operation signal to the controller 51.

The display 53 displays an operation screen, an operating condition of the body 1, and the like, on the display in accordance with display control by the controller 51.

The communication unit 54, which includes a communication interface, performs communication with external equipment on a network.

The storage 55, which includes a non-volatile semiconductor memory, a hard disk, and the like, stores a program to be executed by the controller 51, data necessary for execution of the program, imaging history, and the like.

Note that the imaging history is associated with imaging conditions, subject material information/molding conditions, a parent and child relationship of the subject (a child sample which is part of a parent sample, with respect to the parent sample), environment information such as a temperature/vibration/air pressure at the time of imaging, various kinds of examination data (such as in-situ and an ash measurement result), and the like. Further, the imaging history and the associated information may be recorded in an image header, and the like, and then recorded in the storage 55. Further, in the storage 55, a database (DB) may be provided separately from the controller 5, and the imaging history and the associated information may be managed in the DB.

(Imaging using Talbot interferometer, Talbot-Lau interferometer)

Here, an imaging method using the Talbot interferometer and the Talbot-Lau interferometer will be described.

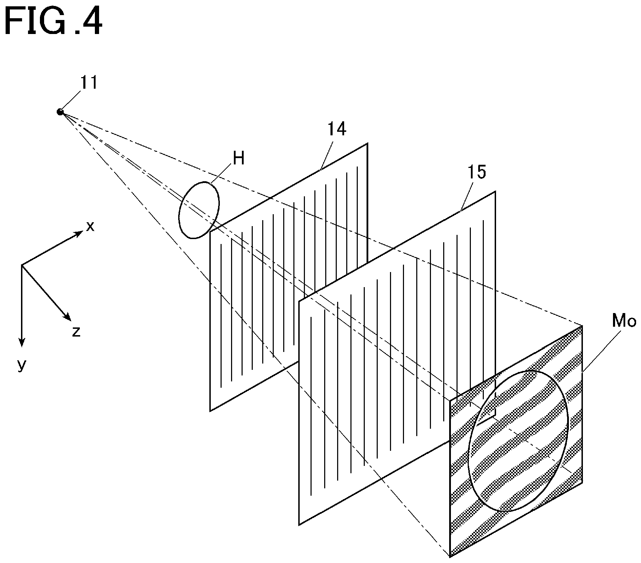

As illustrated in FIG. 4, if an X-ray radiated from the radiation source 11 passes through the first grating 14, the X-ray that has passed forms an image at fixed intervals in the z direction. This image will be referred to as a self-image, and a phenomenon that the self-image is formed will be referred to as a Talbot effect. The second grating 15 is disposed substantially parallel to the self-image at a position at which the self-image is formed, and a Moire fringe image (indicated with Mo in FIG. 4) can be obtained with the X-ray that has passed through the second grating 15. In other words, the first grating 14 forms a periodic pattern, and the second grating 15 converts the periodic pattern to a Moire fringe. If the subject H exists between the radiation source 11 and the first grating 14, a phase of the X-ray is shifted by the subject H, and thus, the Moire fringe on the Moire fringe image becomes disordered from a boundary of a periphery of the subject H as illustrated in FIG. 4. A subject image can be obtained by detecting the disorder of the Moire fringe by processing the Moire fringe image. This is principle of the Talbot interferometer.

In the body 1, the multi-slit 12 is disposed at a position which is between the radiation source 11 and the first grating 14 and which is close to the radiation source 11, and X-ray imaging is performed using the Talbot-Lau interferometer. While it is assumed in the Talbot interferometer that the radiation source 11 is an ideal point source, in actual imaging, a focus having a focal diameter that is large to some extent is used, and thus, an effect of X-rays being radiated as if a plurality of point sources continued is provided by the multi-slit 12. This is an X-ray imaging method by the Talbot-Lau interferometer, and a Talbot effect similar to the effect of the Talbot interferometer can be obtained even in a case where a focal diameter is large to some extent.

In the body 1 of the present embodiment, a Moire fringe image necessary for generating a reconstructed image of the subject H is captured using a fringe scanning method. Fringe scanning typically refers to performing imaging M times (imaging of M steps) (M is a positive integer, M > 2 in the absorption image, M > 3 in a differential phase image and a small-angle scattering image) by relatively moving one (in the present embodiment, the second grating 15) or two of the gratings (the multi-slit 12, the first grating 14, the second grating 15) in the slit period direction (x direction) and acquiring M Moire fringe images necessary for generating a reconstructed image. Specifically, if the slit period of the grating to be moved is set at d ($\mu$m), M Moire fringe images are acquired by repeating imaging while moving the grating in the slit period direction by d/M ($\mu$m) each time.

The reconstructed image to be generated on the basis of the Moire fringe image includes a small-angle scattering image, a differential phase image, and an absorption image.

The small-angle scattering image is an image of scattering of X-rays in a fine structure and has a signal value that becomes greater as scattering of X-rays becomes larger. In the small-angle scattering image, a fine structure aggregate of several micrometers to several tens of micrometers smaller than a pixel size can be grasped.

The differential phase image is an image of refraction of the X-ray by the subject and has a signal value that becomes greater as refraction of the X-ray becomes greater. While lighter elements have lower sensitivity in the absorption image, in the differential phase image, light elements can maintain high sensitivity, so that change of a substance that is difficult to be grasped in the absorption image can be grasped.

The absorption image is an image of absorption of the X-ray by the subject and is equivalent to a plain X-ray image in related art.

In generation of the reconstructed image, for example, first, offset correction processing, gain correction processing, defect pixel correction processing, X-ray intensity fluctuation correction, and the like, are performed on a subject Moire fringe image (Moire fringe image including the subject). Then, the reconstructed image is generated on the basis of the corrected subject Moire fringe image and a back ground (BG) Moire fringe image for_ generating the reconstructed image. The BG Moire fringe image is a Moire fringe image acquired by moving the second grating 15 under the same X-ray irradiation conditions (a tube voltage, an mAS value, a filter) as the conditions for the subject Moire fringe image in a state where the subject H is removed from the subject stand 13.

The absorption image is generated by logarithmically converting a transmittance image generated by dividing an added image of M subject Moire fringe images by an added image of M BG Moire fringe images.

The differential phase image is generated by generating a differential phase image including the subject and a differential phase image without the subject by calculating a phase of Moire fringe using principle of a fringe scanning method for each of the subject Moire fringe image and the BG Moire fringe image and subtracting the generated differential phase image without the subject from the generated differential phase image including the subject.

The small-angle scattering image is generated by generating a small-angle scattering image including the subject and a small-angle scattering image without the subject by calculating visibility of the Moire fringe (visibility = amplitude ÷ average value) using the principle of the fringe scanning method for each of the subject Moire fringe image and the BG Moire fringe image and dividing the generated small-angle scattering image including the subject by the generated small-angle scattering image without the subject.

(Operation of X-ray Talbot imaging apparatus 100, Example 1)

Operation of the X-ray Talbot imaging apparatus 100 will be described next.

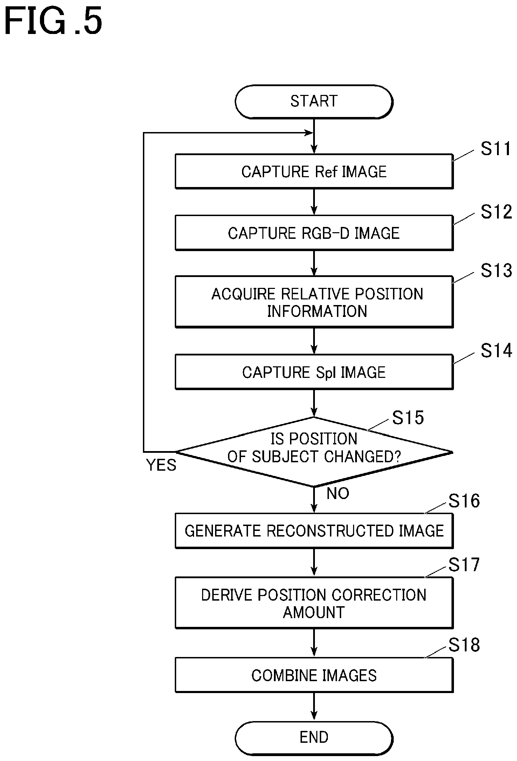

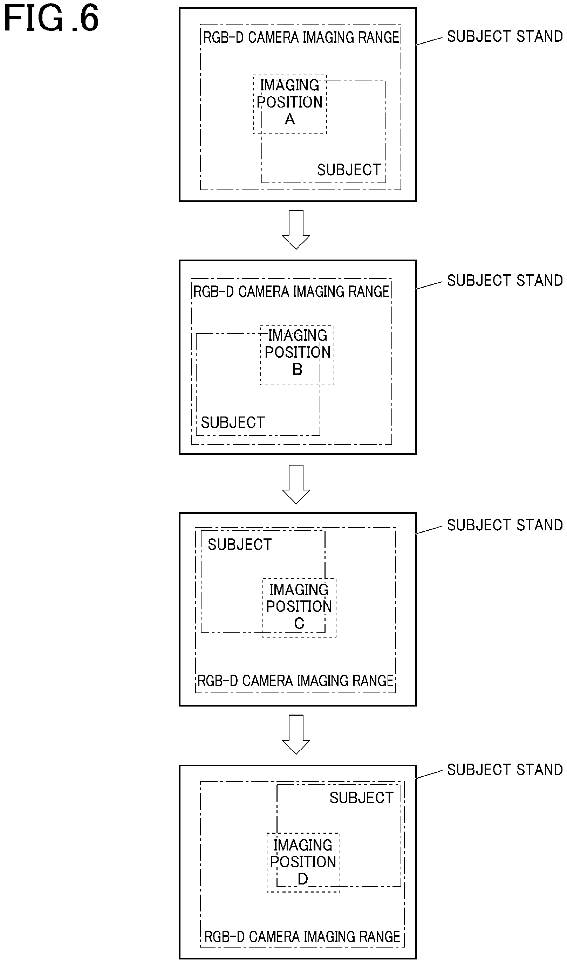

FIG. 5 is a flowchart illustrating imaging processing a to be executed by cooperation of the controller 51 of the X-ray Talbot imaging apparatus 100 and the program stored in the storage 55. As illustrated in FIG. 6, the imaging processing a is processing of generating an image of a range larger than the imaging range of one time by moving the subject H to four positions, performing imaging at imaging positions A to D and combining reconstructed images (the small-angle scattering image, the differential phase image, the absorption image) generated for each of the imaging positions A to D.

If the imaging processing a is started, the controller 51 acquires M BG Moire fringe images (Ref images) without the subject by moving the second grating 15 under the same X-ray irradiation conditions (a tube voltage, an mAs value, a filter) as the conditions for the subject Moire fringe image in a state where the subject H is not disposed on the subject stand 13 (step S11). Note that it is assumed in Example 1 that four images are acquired.

After the BG Moire fringe images (Ref images) are acquired, a user disposes the subject H on the subject stand 13.

Then, the controller 51 causes the camera 21 to image the subject H to acquire an RGB-D image (step S12).

Then, the controller 51 acquires relative position information that is relative positions in two or more dimensions of the subject H with respect to the gratings from the RGB-D image (step S13).

Then, the controller 51 controls the radiation source 11, the movement mechanism 15a, the radiation detector 16, and the like, to perform imaging (referred to as Talbot imaging) for acquiring Moire fringe images (Spl images) by utilizing the above-described Talbot effect (step S14). In the present embodiment, M (four) subject Moire fringe images (Spl images) are acquired while moving the grating (in the present embodiment, the second grating 15) in the slit period direction by the fringe scanning method.

Then, the controller 51 determines whether or not the position of the subject H is changed (step S15). In Example 1, imaging is performed at four locations of the imaging positions A to D illustrated in FIG. 6, and thus, four loops of the processing from step S11 to step S14 are performed. Thus, the controller 51 repeats change of the imaging position four times and then determines that the position of the subject H is not changed (step S15: No), and the processing proceeds to step S16. Further, in a case where the controller 51 determines that the position of the subject H is changed (step S15: Yes), the user moves the subject H from the subject stand 13, and the processing proceeds to step S11. Note that in a case where the controller 51 determines that the position of the subject H is changed (step S15: Yes), the processing may proceed to step S12 without acquiring the BG Moire fringe images again. Note that while the subject H may be manually moved from the subject stand 13, a motorized stage is preferably utilized in terms of movement accuracy.

Then, the reconstructed images (the small-angle scattering image, the differential phase image, the absorption image) are generated on the basis of the BG fringe Moire images and the subject Moire fringe images at the respective imaging positions (step S16). In Example 1, four images are generated. The reconstructed images are stored in the storage 55 in association with the relative position information at the respective imaging positions, image numbers for identifying the images, test conditions, imaging conditions in Talbot imaging, imaging date and time, and the like.

Then, the controller 51 derives a position correction amount on the basis of the relative position information at the respective imaging positions (step S17). Derivation of the position correction amount will be described later. For example, a gradient descent method can be used. Note that in a case where the subject is thick, influence of oblique incidence of the X-ray cannot be ignored, and thus, this influence is taken into account.

Finally, the controller 51 corrects displacement of the reconstructed images at the respective positions on the basis of the position correction amount derived in step S17 and combines the reconstructed images at the respective positions (step S18). Note that in a case where the position of the subject H is never moved, the processing may be finished without executing step S17 and step S18. Further, the displacement may be corrected for the subject Moire fringe image before the reconstructed image is created. This method has higher accuracy.

Thus, by using the relative positions in two or more dimensions of the subject H with respect to the gratings, the positions can be aligned with high accuracy, and an image of a range larger than the imaging range of one time can be generated. Further, the positions can be aligned without using the marker, and thus, a defect portion does not occur.

(Example 2)

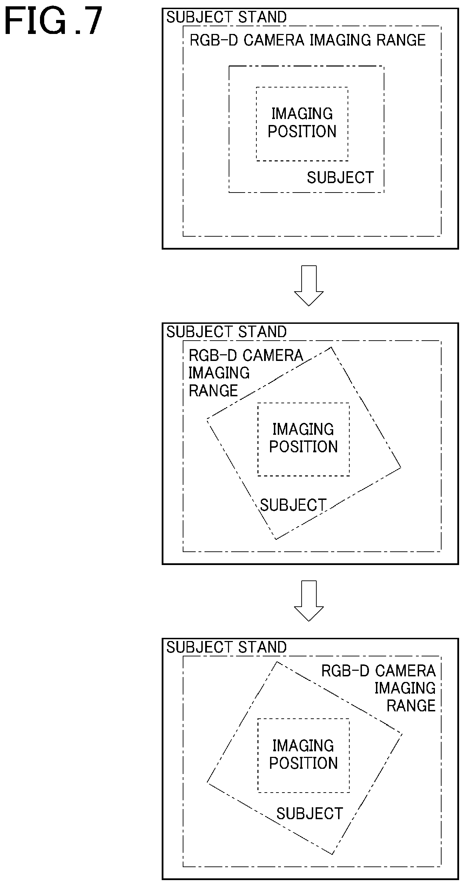

Example 2 is processing of generating a small-angle scattering image by performing imaging while moving the subject H to three imaging positions (having relative angles of 0°, 60°, 120° with respect to the grating) as illustrated in FIG. 7 and combining the reconstructed images (the small-angle scattering images) generated for each imaging position. Note that flow of the processing is the same as the flow in Example 1, and the controller 51 generates a small-angle scattering oriented image in step S18.

Note that the small-angle scattering oriented image is an image representing orientation of a substance such as a fiber inside the subject H obtained by analyzing a plurality of small-angle scattering images generated on the basis of the Moire fringe images captured while setting three or more relative angles between the subject H and the grating around the radiation irradiation axis.

While in the example illustrated in FIG. 7, three imaging positions having relative angles of 0°, 60° and 120° with respect to the gratings are illustrated, the present invention is not limited to this example. The relative angles with respect to the gratings are determined by the number of times of imaging (N) and determined on the basis of the following expression (1).

$$\text{Relative angle} = 180°/N \text{ (where N is an integer equal to or greater than 3) ... expression (1)}$$

Thus, by using the relative positions in two or more dimensions of the subject H with respect to the gratings, it is possible to generate the small-angle scattering oriented image while aligning the positions with high accuracy. Further, correction can be performed without providing markers, so that a defect portion does not occur. Further, displacement can be corrected while the center of rotation of the subject is grasped, so that, by returning the rotation around the center of a pixel corresponding to the center of the rotation of the subject, correction can be performed with higher accuracy compared to a case where displacement is corrected while the rotation is returned around the center of the image.

Note that in a case where displacement is corrected from the image, which pixel actually corresponds to the center of the rotation is unknown, and thus, it is necessary to return the rotation around the center of rotation (normally the center of the image) as design of the subject stand 13 of the X-ray Talbot imaging apparatus 100, which causes an error.

(Example 3)

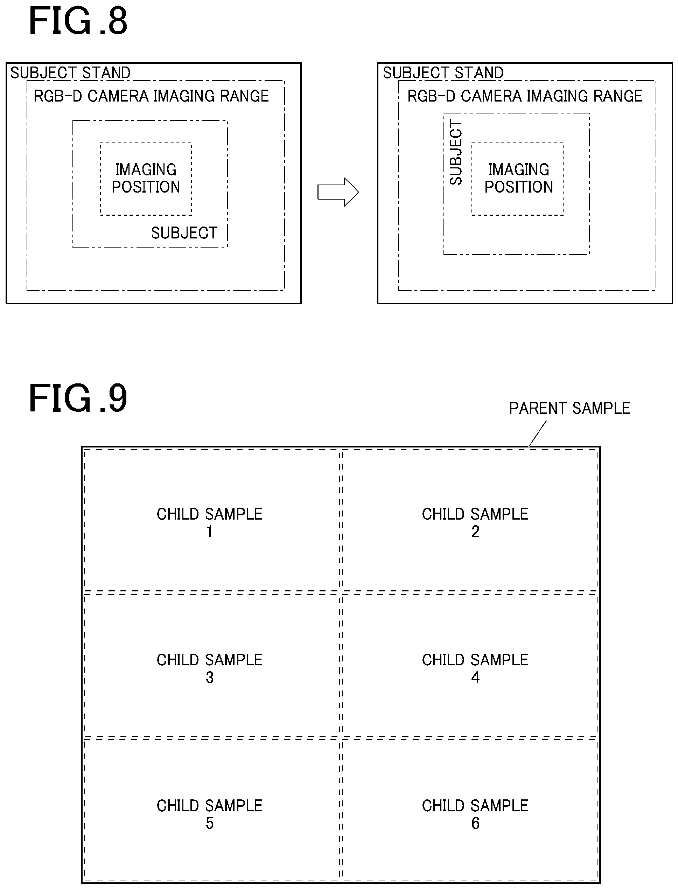

Example 3 is processing of generating the reconstructed images (the small-angle scattering image, the differential phase image, the absorption image) by performing imaging while moving the subject H to two imaging positions (having relative angles of 0° and 90° with respect to the grating) as illustrated in FIG. 8 and combining the reconstructed images (the small-angle scattering image, the differential phase image, the absorption image) generated for each imaging position. Note that flow of the processing is the same as the flow in Example 1, and the controller 51 generates the combined reconstructed image (the small-angle scattering image, the differential phase image, the absorption image) in step S18.

In the example illustrated in FIG. 8, in image combining in step S18, specifically, a value of each pixel is obtained by using an average value of each pixel and a square-root of sum of squares of each pixel for each pixel as in the following expression. Particularly, in the small-angle scattering image and the differential phase image, influence by orientation of the subject H with respect to the grating can be reduced.

[Math. 1]

$$\text{Absorption: } (0°\text{ Image} + 90°\text{ Image})/2$$
$$\text{Small-angle : } (0°\text{ Image} + 90°\text{ Image})/2$$

$$\text{Differential phase: } (0°\text{ Image} + 90°\text{ Image}) \text{ or } \sqrt{(0°\text{ Image}^2 + 90°\text{ Image}^2)}$$

Thus, by using the relative positions in two or more dimensions of the subject H with respect to the gratings, it is possible to generate the small-angle scattering image, the differential phase image and the absorption image while aligning the positions with high accuracy. Further, correction can be performed without providing a marker, so that a defect portion does not occur. Further, displacement can be corrected while the center of rotation of the subject is grasped, so that the positions can be aligned by returning the rotation on the basis of the center of the image, which means that displacement can be corrected with higher accuracy than displacement correction in the image.

(Example 4)

Example 4 is processing of generating reconstructed images (the small-angle scattering image, the differential phase image, the absorption image) of a parent sample by combining the reconstructed images (the small-angle scattering image, the differential phase image, the absorption image) obtained by imaging the subject H (parent sample) for each of six child samples as illustrated in FIG. 9 on the basis of position information (relative positions in two or more dimensions of the subject H with respect to the gratings) of the child samples with respect to the parent sample. Note that flow of the processing is the same as the flow in Example 1, and the controller 51 generates the reconstructed images (the small-angle scattering image, the differential phase image, the absorption image) of the parent sample in step S18. Further, it is necessary to cut out the child samples from the parent sample and manage a parent and child relationship of the samples and cut-out positions in the DB.
Thus, a state of the parent sample can be restored with high accuracy from images of a plurality of child samples.

(Example 5)

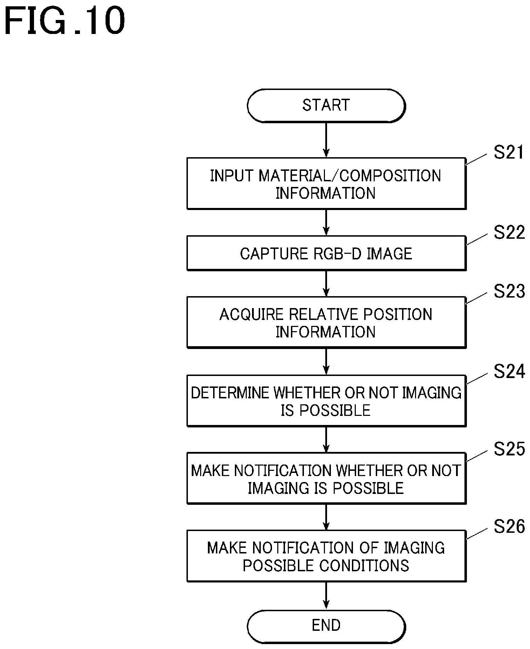

FIG. 10 is a flowchart illustrating imaging possible/impossible determination processing b to be executed by cooperation of the controller 51 of the X-ray Talbot imaging apparatus 100 and the program stored in the storage 55. The imaging possible/impossible determination processing b is processing of determining whether or not imaging is possible from material and composition information input using the operator 52, thickness information of the subject H obtained from the RGB-D image, past imaging information, and current imaging conditions.
First, the controller 51 obtains material and composition information of the subject H via the operator 52 (step S21).
Then, the controller 51 causes the camera 21 to image the subject H to acquire the RGB-D image (step S22).
Then, the controller 51 acquires relative position information (particularly, thickness information) that is relative positions in two or more dimensions of the subject H with respect to the gratings from the RGB-D image (step S23).
Then, the controller 51 determines whether or not imaging is possible by matching the current imaging conditions with reference to imaging conditions, and the like, of a similar subject imaged in the past stored in the storage 55 from the material and composition information and the thickness information (step S24). Note that imaging possible conditions may be derived.
Specifically, there can be a case where the subject H is a thick aluminum plate. In this case, if the X-ray radiated from the radiation source 11 is weak, the X-ray does not pass through the subject H, and thus, imaging cannot be performed. Thus, in step S24, the controller 51 determines that imaging is impossible. Further, the controller 51 derives setting (mAs value) of the radiation source 11 in accordance with the thickness.
Note that while in the above description, whether or not imaging is possible is determined by referring to the past imaging information, the present invention is not limited to this. For example, whether or not imaging is possible may be determined by calculating a transmission amount of the X-ray on the basis of physical information of the subject H.
Then, the controller 51 makes an imaging possible/impossible notification via the display 53 (step S25). Finally, the controller 51 makes a notification of imaging possible conditions via the display 53 (step S26).

Note that a notification method is not limited to screen display and may be sound, speech or LED display. It is also possible to employ a configuration where imaging is not started in a case where it is determined that imaging is impossible. Further, it is also possible to employ a configuration where, when an instruction to start imaging is issued, in a case where a favorable image cannot be obtained even if imaging is performed, imaging is allowed after a notification indicating that a favorable image cannot be obtained is made. Further, in a case where imaging is possible, the processing may be finished without making an imaging possible/impossible notification (step S25) and making a notification of imaging possible conditions (step S26).

The user can therefore grasp conditions for being able to capture an image having necessary drawing performance (such as contrast) with high accuracy. Further, it is possible to reduce work for determining whether or not imaging is possible.

(Derivation of position correction amount)

The derivation of the position correction amount to be executed by the controller 51 in step S17 in FIG. 5 will be described on the basis of imaging (Example 3) of the subject H at the two imaging positions (having relative angles of 0° and 90° with respect to the gratings).

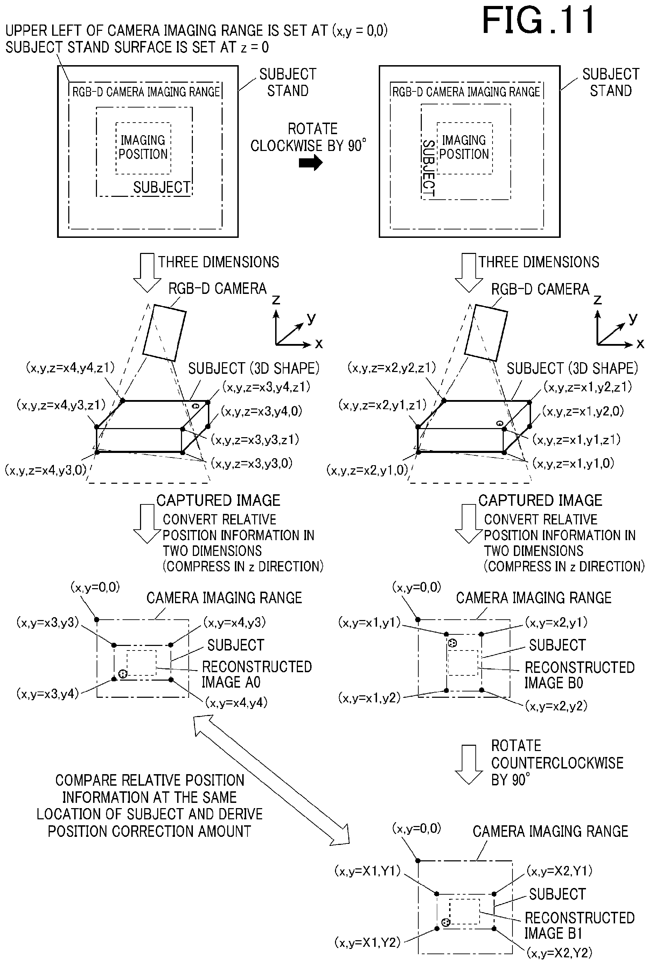

As in the upper part of FIG. 11, two imaging positions obtained by rotating the subject by 90° around the radiation irradiation axis are used.

The second part from the top in FIG. 11 indicates relative position information of the subject H in a case where the subject is considered in three dimensions at the two imaging positions. Further, a circle on the subject H is a marker. It can be known from the right and left figures that the subject H is rotated by 90° around the radiation irradiation axis. Note that the marker is provided for facilitating understanding of description and the drawings, and this marker is not necessary in the present invention.

The third part from the top in FIG. 11 is relative position information of the reconstructed images (a reconstructed image A0, a reconstructed image B0) and the subject H at the two imaging positions. The reconstructed images have two dimensions, and thus, are compressed in the z direction to convert the three-dimensional relative position information into two-dimensional relative position information.

The fourth part from the top in FIG. 11 is relative position information of a reconstructed image B1 and the subject H obtained by rotating by -90° the reconstructed image of the subject H at the imaging position rotated around the radiation irradiation axis by 90°.

The position correction amount is derived by comparing the relative position information at the same location of the subject H in the reconstructed image A0 and the reconstructed image B1. The position correction amount is derived in a parallel direction (Xcor, Ycor) and in a rotation direction (ΘCor).

For example, Xcor, Ycor and 0Cor such that diff becomes a minimum are derived using the gradient descent method, and displacement correction (parallel movement and rotational movement) is applied to the reconstructed image A0 or the reconstructed image B1.

$$\text{diff} = |\,x3 - X1\,| + |\,x4 - X2\,| + |\,y3 - Y1\,| + |\,y4 - Y2\,| \qquad \text{... expression (2)}$$

Note that methods other than the gradient descent method may be used.

[Second embodiment]

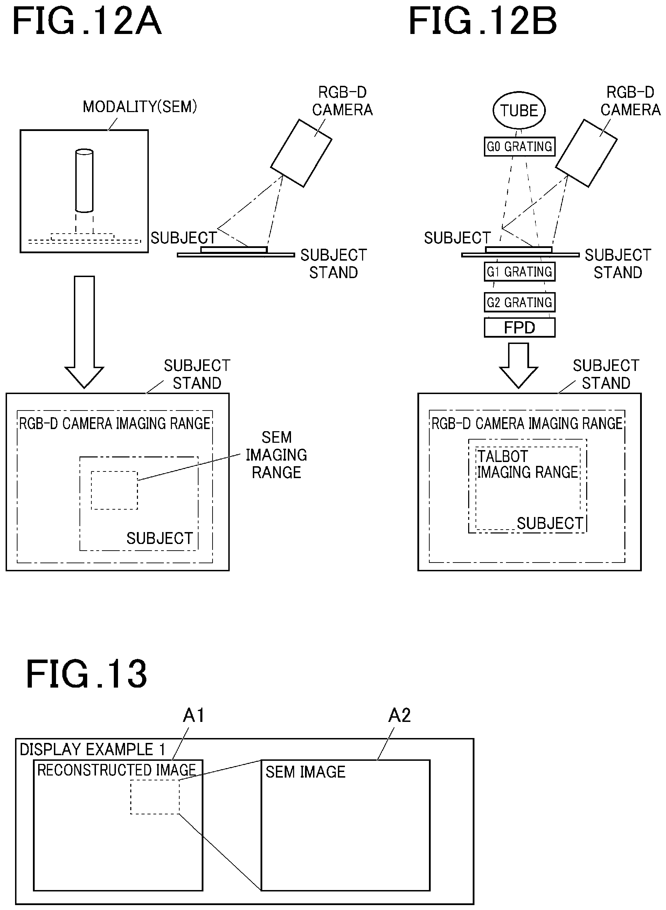

In a second embodiment, as illustrated in FIG. 12A and FIG. 12B, imaging is performed using an RGB-D camera also in imaging using other modality in a similar manner in addition to the first embodiment. Further, the controller 51 acquires a captured image and relative position information (acquired from the RGB-D image in other modality) from the other modality and causes a subject image of the other modality to match a subject image of the X-ray Talbot imaging apparatus 100 on the basis of the relative position information. The other modality only requires to be modality that outputs image data from which an imaging range and a subject can be grasped in the same coordinate system and can include, for example, SEM, μCT and a high-resolution Talbot.

In other words, the controller 51 functions as an acquirer that acquires relative position information (relative position information acquired in the other modality, and relative position information in imaging using the X-ray Talbot imaging apparatus 100) that is relative positions in two or more dimensions of the subject with respect to the gratings.

FIG. 13 is an example in which the reconstructed image A1 obtained by the X-ray Talbot imaging apparatus 100 and an image A2 obtained by other modality (SEM) are displayed side by side on the display 53. A position coordinate of the reconstructed image A1 corresponds to a position coordinate of the image A2, and thus, the image A2 corresponding to part of the reconstructed image A1 can be displayed.

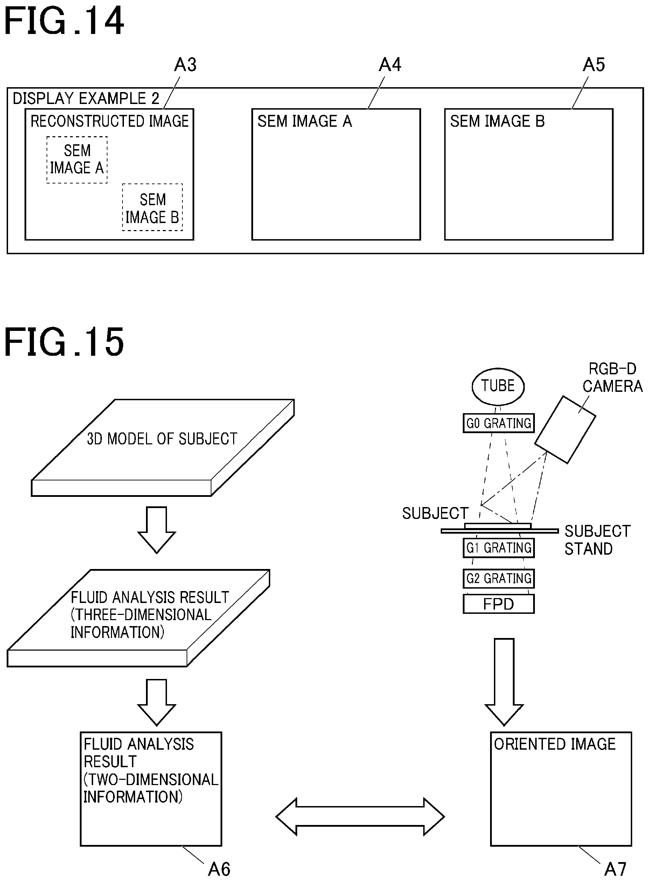

FIG. 14 is an example in which a reconstructed image A3 obtained by the X-ray Talbot imaging apparatus 100 and an image A4 and an image A5 obtained by other modality (SEM) are displayed side by side on the display 53. A position coordinate of the reconstructed image A3 corresponds to position coordinates of the image A4 and the image A5, and thus, the image A4 and the image A5 corresponding to part of the reconstructed image A3 can be displayed.

Thus, by using the relative positions in two or more dimensions of the subject H with respect to the gratings, it is possible to align the positions with high accuracy and make positions match among images of two or more different kinds of modality. Further, the positions can be aligned without using a marker, so that a defect portion does not occur.

[Third embodiment]

In a third embodiment, as illustrated in FIG. 15, data in a format that can be fed back to computer-aided engineering (CAE) is generated by the controller 51 making coordinates of the reconstructed images (the small-angle scattering image, the differential phase image, the absorption image) obtained by the X-ray Talbot imaging apparatus 100 match coordinates of an image created by a CAD on the basis of the relative positions in two or more dimensions of the subject H with respect to the gratings obtained from the RGB-D image by using the CAD in addition to the first embodiment.

In other words, the controller 51 functions as an acquirer that acquires relative position information (coordinate information of the image created by the CAD and relative position information obtained from the RGB-D image) that is relative positions in two or more dimensions of the subject with respect to the gratings.

In the example in FIG. 15, by obtaining a fluid analysis result of three-dimensional information from a 3D model of the subject created by the CAD and compressing (integrating in view of vectors) information in the Z direction, a fluid analysis result A6 of a two-dimensional image can be obtained. A position of the fluid analysis result A6 of the two-dimensional image is made to match a position of the reconstructed image A7 obtained by the X-ray Talbot imaging apparatus 100.

Thus, by using the relative positions in two or more dimensions of the subject H with respect to the grating, it is possible to align the positions with high accuracy and make positions match the positions in the image obtained from the CAD. Further, the positions can be aligned without using a marker, so that a defect portion does not occur.

Particularly, the third embodiment is useful because the fluid analysis result and an oriented image can be accurately compared.

[Other embodiments]

Note that the reconstructed image and the RGB-D image can be displayed in an overlay manner. This can allow confirmation while comparing the images.

Further, by using the imaging history and the associated information stored in the storage 55, it is possible to align the positions with further higher accuracy. Particularly, the positions can be aligned with higher accuracy by grasping a state of the subject H from a relationship between environment information such as a temperature/vibration/air pressure at the time of imaging and various kinds of examination data (such as in-situ and an ash measurement result).

As described above, as a result of the radiographic imaging apparatus in which a radiation source, a plurality of gratings and a radiation detector are provided side by side in a radiation irradiation axis direction, including a generator (controller 51) that generates a reconstructed image on the basis of a Moire fringe image obtained by performing imaging by irradiating a subject disposed at a position overlapping the radiation irradiation axis direction with radiation by the radiation source, an acquirer (controller 51) that acquires relative position information that is relative positions in two or more dimensions of the subject with respect to the gratings, a controller (controller 51) that associates the reconstructed image with the relative position information, it is possible to align positions of images obtained by imaging a plurality of times, the subject that has no significant characteristics on the images and has a size exceeding the imaging range of one time, with high accuracy without displacement.

Note that the acquirer (controller 51) may acquire a captured image and relative position information acquired by modality different from the radiographic imaging apparatus.

Further, the acquirer may acquire relative position information from an image captured with an imager that captures an image from which the relative position information can be acquired.

Further, the generator generates a plurality of reconstructed images and generates a combined image by correcting positions of the plurality of reconstructed images using the relative position information, so that it is possible to align positions of images obtained by imaging a plurality of times, a subject that has no significant characteristics on the images and has a size exceeding an imaging range of one time, with high accuracy without displacement and combine the images with high accuracy.

Further, the generator generates a reconstructed image of the parent sample by combining reconstructed images of the subject cut out from the same parent sample using the relative position information of the subject cut out from the parent sample, so that it is possible to align positions of images obtained by imaging a plurality of times, a subject that has no significant characteristics on the images and has a size exceeding an imaging range of one time, with high accuracy without displacement and combine the images with high accuracy.

Further, the generator generates a plurality of reconstructed images, which are small-angle scattering images, and generates an oriented image using the small-angle scattering images at three or more angles rotated around the radiation irradiation axis, for which positions are corrected using the relative position information, so that it is possible to align positions of images obtained by imaging a plurality of times, a subject that has no significant characteristics on the images and has a size exceeding an imaging range of one time, with high accuracy without displacement and obtain an oriented image with high accuracy.

Further, the generator generates a plurality of reconstructed images, which are small-angle scattering images or differential phase images, and generates an image by synthesizing the small-angle scattering images or the differential phase images at two or more angles rotated around the radiation irradiation axis, for which positions are corrected using the relative position information, so that it is possible to align positions of images obtained by imaging a plurality of times, a subject that has no significant characteristics on the images and has a size exceeding an imaging range of one time, with high accuracy without displacement and combine the images with high accuracy.

Further, the generator associates the relative position information with coordinate information of a computer-aided design (CAD) model and generates data in a format that can be fed back to computer-aided engineering (CAE), so that it is possible to align positions of a CAD image and the reconstructed image with high accuracy without displacement.

Further, the controller determines whether or not imaging is possible from the material and/or composition information of the subject and the thickness information of the subject obtained from the relative position information, so that the user can reduce work for determining whether or not imaging is possible.

Further, the controller derives imaging possible conditions from the material and/or composition information of the subject and the thickness information of the subject obtained from the relative position information, so that the user can set appropriate imaging possible conditions by the imaging possible conditions being derived.

Further, an image generation method is a method to be performed by a radiographic imaging apparatus in which a radiation source, a plurality of gratings and a radiation detector are provided side by side in a radiation irradiation axis direction, the method including generating by a generator, a reconstructed image on the basis of a Moire fringe image obtained by performing imaging by irradiating a subject disposed at a position overlapping the radiation irradiation axis direction with radiation by the radiation source, acquiring by an acquirer, relative position information that is relative positions in two or more dimensions of the subject with respect to the gratings, and associating by a controller, the reconstructed image with the relative position information, so that it is possible to align positions and combine images obtained by imaging a plurality of times, a subject that has no significant characteristics on the images and has a size exceeding an imaging range of one time, with high accuracy without displacement to generate the reconstructed image.

Further, a radiographic imaging system is a radiographic imaging system including the above-described radiographic imaging apparatus, and at least one apparatus of $\mu$CT, SEM or high-resolution Talbot and matches positions of an analysis result of the apparatus and the reconstructed image using the relative position information, so that it is possible to align positions of the analysis result of the other apparatus and the reconstructed image obtained from the radiographic imaging apparatus with high accuracy without displacement.

Further, the radiographic imaging system includes the above-described radiographic imaging apparatus and displays the reconstructed image and an optical image obtained by an imager in a superimposed manner using the relative position information, so that it is possible to superimpose the images without displacement.

Further, a program causes a computer of a radiographic imaging apparatus in which a radiation source, a plurality of gratings and a radiation detector are provided side by side in a radiation irradiation axis direction to function as a generator that generates a reconstructed image on the basis of a Moire fringe image obtained by performing imaging by irradiating a subject disposed at a position overlapping the radiation irradiation axis direction with radiation by the radiation source, an acquirer that acquires relative position information that is relative positions in two or more dimensions of the subject with respect to the gratings, and a controller that associates the reconstructed image with the relative position information, so that it is possible to align positions of images obtained by imaging a plurality of times, a subject that has no significant characteristics on the images and that has a size exceeding an imaging range of one time, with high accuracy without displacement.

While the first to the second embodiments of the present invention and the modifications thereof have been described above, the above description in the embodiments is merely a preferred example according to the present invention, and the present invention is not limited to these.

For example, while an example of a test apparatus using a Talbot-Lau interferometer of a scheme in which the second grating 15 is moved with respect to the multi-slit 12 and the first grating 14 at the time of imaging using the fringe scanning method has been described in the above-described embodiment, the present invention may be applied to a test apparatus using a Talbot-Lau interferometer of a scheme in which one or two grating of the multi-slit 12, the first grating 14 or the second grating 15 is moved at the time of imaging using the fringe scanning method. Further, the present invention may be applied to a test apparatus using a Talbot interferometer of a scheme in which one of the first grating 14 or the second grating 15 is moved with respect to the other grating. Further, the present invention may be applied to a test apparatus using a Talbot-Lau interferometer of a scheme in which one of the multi-slit 12 or the first grating 14 is moved with respect to the other grating. Further, the present invention may be applied to a Talbot-Lau interferometer, a Talbot interferometer, or a Talbot-Lau interferometer using a Fourier transform method that does not require fringe scanning.

Further, while a case has been described as an example in the above-described embodiment where a small-angle scattering image, a differential phase image and an absorption image are generated on the basis of the Moire fringe image obtained through Talbot imaging, the present invention can be implemented if the small-angle scattering image and/or the differential phase image can be generated.

Further, for example, while an example has been described above where a hard disk, a non-volatile memory of a semiconductor, or the like, is used as a computer-readable medium for the program according to the present invention, the present invention is not limited to this example. As another computer-readable medium, a portable recording medium such as a CD-ROM can be applied. Further, a carrier wave can be applied as a medium that provides data of the program according to the present invention via a communication line.

Although embodiments of the present invention have been described and illustrated in detail, the disclosed embodiments are made for purposes of illustration and example only and not limitation. The scope of the present invention should be interpreted by terms of the appended claims.

**Claims**

1. A radiographic imaging apparatus (100) in which a radiation source (11), a plurality of gratings (14, 15) and a radiation detector (16) are provided side by side in a radiation irradiation axis direction, the radiographic imaging apparatus comprising:

   a generator (51) that generates a reconstructed image on a basis of a Moire fringe image obtained by performing imaging by irradiating a subject disposed at a position overlapping the radiation irradiation axis direction with radiation by the radiation source (11);
   an acquirer (51) that acquires relative position information that is relative positions in two or more dimensions of the subject with respect to the gratings (14, 15); and
   a controller (51) that associates the reconstructed image with the relative position information.

2. The radiographic imaging apparatus (100) according to claim 1,
   wherein the acquirer (51) acquires the relative position information from an image captured by an imager (21) that captures an image from which the relative position information can be acquired.

3. The radiographic imaging apparatus (100) according to claim 1 or 2,

   wherein the generator (51) generates a plurality of reconstructed images, and
   the generator (51) generates a combined image by correcting positions of the plurality of reconstructed images using the relative position information.

4. The radiographic imaging apparatus (100) according to any one of claims 1 to 3,
   wherein the generator (51) combines the reconstructed images of the subject cut out from a same parent sample using the relative position information of the subject cut out from the parent sample to generate the reconstructed image of the parent sample.

5. The radiographic imaging apparatus (100) according to claim 1 or 2,

   wherein the generator (51) generates a plurality of reconstructed images,
   the reconstructed images are small-angle scattering images, and
   the generator (51) generates an oriented image using the small-angle scattering images at three or more angles rotated around the radiation irradiation axis, for which positions are corrected using the relative position information.

6. The radiographic imaging apparatus (100) according to claim 1 or 2,

wherein the generator (51) generates a plurality of reconstructed images,
the reconstructed images are small-angle scattering images or differential phase images, and
the generator (51) generates an image by synthesizing the small-angle scattering images or the differential phase images at two or more angles rotated around the radiation irradiation axis, for which positions are corrected using the relative position information.

7. The radiographic imaging apparatus (100) according to any one of claims 1 to 6,
wherein the generator (51) associates the relative position information with coordinate information of a computer-aided design (CAD) model to generate data in a format that can be fed back to computer-aided engineering (CAE).

8. The radiographic imaging apparatus (100) according to any one of claims 1 to 7,
wherein the controller (51) determines whether or not imaging is possible from material and/or composition information of the subject and thickness information of the subject obtained from the relative position information.

9. The radiographic imaging apparatus (100) according to any one of claims 1 to 8,
wherein the controller (51) derives imaging possible conditions from material and/or composition information of the subject and thickness information of the subject obtained from the relative position information.

10. An image generation method to be performed by a radiographic imaging apparatus (100) in which a radiation source (11), a plurality of gratings (14, 15) and a radiation detector (16) are provided side by side in a radiation irradiation axis direction, the image generation method comprising:

generating, by a generator (51), a reconstructed image on a basis of a Moire fringe image obtained by performing imaging by irradiating a subject disposed at a position overlapping the radiation irradiation axis direction with radiation by the radiation source (11);
acquiring, by an acquirer (51), relative position information that is relative positions in two or more dimensions with respect to the gratings (14, 15); and
associating, by a controller (51), the reconstructed image with the relative position information.

11. A radiographic imaging system comprising:

the radiographic imaging apparatus (100) according to any one of claims 1 to 9; and
an apparatus that outputs image data from which an imaging range and the subject can be grasped in the same coordinate system,
wherein positions of an analysis result of the apparatus are made to match positions of the reconstructed image using the relative position information.

12. A radiographic imaging system comprising the radiographic imaging apparatus (100) according to claim 2,
wherein positions of the reconstructed image and an optical image obtained by the imager (21) are aligned using the relative position information, and the reconstructed image and the optical image are displayed in a superimposed manner.

13. A program causing a computer of a radiographic imaging apparatus (100) in which a radiation source (11), a plurality of gratings (14, 15) and a radiation detector (16) are provided side by side in a radiation irradiation axis direction, to function as:

a generator (51) that generates a reconstructed image on a basis of a Moire fringe image obtained by performing image by irradiating a subject disposed at a position overlapping the radiation irradiation axis direction with radiation by the radiation source (11);
an acquirer (51) that acquires relative position information that is relative positions in two or more dimensions of the subject with respect to the gratings (14, 15); and
a controller (51) that associates the reconstructed image with the relative position information.

**Patentansprüche**

1. Ein radiographisches Abbildungsgerät (100), in dem eine Strahlungsquelle (11), eine Vielzahl von Gittern (14, 15) und ein Strahlungsdetektor (16) nebeneinander in einer Strahlungsbestrahlungsachsenrichtung vorgesehen sind, wobei das radiographische Abbildungsgerät umfasst:

   einen Erzeuger (51), der ein rekonstruiertes Bild auf der Basis eines Moire-Streifenbildes erzeugt, das durch Ausführen einer Abbildung durch Bestrahlung eines Subjekts mit Strahlung durch die Strahlungsquelle (11) erhalten wurde, wobei das Subjekt an einer Position angeordnet ist, die die Strahlungsbestrahlungsachsenrichtung überlappt;
   einen Erfasser (51), der relative Positionsinformationen erfasst, die relative Positionen in zwei oder mehr Dimensionen des Subjekts in Bezug auf die Gitter (14, 15) sind; und
   eine Steuerung (51), die das rekonstruierte Bild mit den relativen Positionsinformationen verknüpft.

2. Das radiographische Abbildungsgerät (100) gemäß Anspruch 1,
   wobei der Erfasser (51) die relativen Positionsinformationen aus einem Bild erfasst, das von einem Bildgeber (21) aufgenommen wurde, der ein Bild aufnimmt, aus dem die relativen Positionsinformationen erfasst werden können.

3. Das radiographische Abbildungsgerät (100) gemäß Anspruch 1 oder 2,

   wobei der Erzeuger (51) eine Vielzahl von rekonstruierten Bildern erzeugt, und
   der Erzeuger (51) ein kombiniertes Bild erzeugt, indem er Positionen der mehreren rekonstruierten Bilder unter Verwendung der relativen Positionsinformationen korrigiert.

4. Das radiographische Abbildungsgerät (100) gemäß einem der Ansprüche 1 bis 3,
   wobei der Erzeuger (51) die rekonstruierten Bilder des aus einer gleichen Ausgangsprobe ausgeschnittenen Subjekts unter Verwendung der relativen Positionsinformation des aus der Ausgangsprobe ausgeschnittenen Subjekts kombiniert, um das rekonstruierte Bild der Ausgangsprobe zu erzeugen.

5. Das radiographische Abbildungsgerät (100) gemäß Anspruch 1 oder 2,

   wobei der Erzeuger (51) eine Vielzahl von rekonstruierten Bildern erzeugt,
   die rekonstruierten Bilder Kleinwinkelstreuungsbilder sind, und
   der Erzeuger (51) ein orientiertes Bild unter Verwendung der Kleinwinkelstreuungsbilder in drei oder mehr um die Strahlungsbestrahlungsachse gedrehten Winkeln erzeugt, für die Positionen unter Verwendung der relativen Positionsinformationen korrigiert werden.

6. Das radiographische Abbildungsgerät (100) gemäß Anspruch 1 oder 2,

   wobei der Erzeuger (51) eine Vielzahl von rekonstruierten Bildern erzeugt,
   die rekonstruierten Bilder Kleinwinkelstreuungsbilder oder Differentialphasenbilder sind, und
   der Erzeuger (51) ein Bild erzeugt, indem er die Kleinwinkelstreuungsbilder oder die Differentialphasenbilder unter zwei oder mehr um die Strahlungsbestrahlungsachse gedrehten Winkeln synthetisiert, für die Positionen unter Verwendung der relativen Positionsinformationen korrigiert werden.

7. Das radiographische Abbildungsgerät (100) gemäß einem der Ansprüche 1 bis 6,
   wobei der Erzeuger (51) die relativen Positionsinformationen mit Koordinateninformationen eines computergestützten Konstruktionsmodells (CAD) verknüpft, um Daten in einem Format zu erzeugen, das an die computergestützte Konstruktion (CAE) zurückgegeben werden kann.

8. Das radiographische Abbildungsgerät (100) gemäß einem der Ansprüche 1 bis 7,
   wobei die Steuerung (51) anhand von aus den relativen Positionsinformationen gewonnenen Material- und/oder Zusammensetzungsinformationen des Subjekts und Dickeninformationen des Subjekts, bestimmt, ob eine Abbildung möglich ist oder nicht.

9. Das radiographische Abbildungsgerät (100) gemäß einem der Ansprüche 1 bis 8,
   wobei die Steuerung (51) Abbildungs-mögliche Bedingungen aus aus den relativen Positionsinformationen gewonnenen Material- und/oder Zusammensetzungsinformationen des Subjekts und Dickeninformationen des Subjekts ableitet.

10. Ein Verfahren zur Bilderzeugung, das von einem radiographischen Abbildungsgerät (100) durchzuführen ist, in dem eine Strahlungsquelle (11), eine Vielzahl von Gittern (14, 15) und ein Strahlungsdetektor (16) nebeneinander in einer Strahlungsbestrahlungsachsenrichtung vorgesehen sind, das Verfahren zur Bilderzeugung umfassend:

Erzeugen, durch einen Erzeuger (51), eines rekonstruierten Bildes auf der Basis eines Moire-Streifenbildes, das durch Ausführen einer Abbildung durch Bestrahlung eines Subjekts mit Strahlung durch die Strahlungsquelle (11) erhalten wurde, wobei das Subjekt an einer Position angeordnet ist, die die Strahlungsbestrahlungsachsenrichtung überlappt;
Erfassen, durch einen Erfasser (51), von relativen Positionsinformationen, die relative Positionen in zwei oder mehr Dimensionen in Bezug auf die Gitter (14, 15) betreffen; und
Verknüpfen des rekonstruierten Bildes mit den relativen Positionsinformationen durch eine Steuerung (51).

11. Ein radiographisches Abbildungssystem, umfassend:

das radiographische Abbildungsgerät (100) gemäß einem der Ansprüche 1 bis 9; und
eine Vorrichtung, die Bilddaten ausgibt, aus denen ein Abbildungsbereich und das Subjekt im gleichen Koordinatensystem erfasst werden können,
wobei die Positionen eines Analyseergebnisses der Vorrichtung mit den Positionen des rekonstruierten Bildes unter Verwendung der relativen Positionsinformationen in Übereinstimmung gebracht wurden.

12. Ein radiographisches Abbildungssystem umfassend das radiographische Abbildungsgerät (100) gemäß Anspruch 2, wobei Positionen des rekonstruierten Bildes und eines durch den Bildgeber (21) erhaltenen optischen Bildes unter Verwendung der relativen Positionsinformationen ausgerichtet werden und das rekonstruierte Bild und das optische Bild in einer überlagerten Weise angezeigt werden.

13. Ein Programm, das einen Computer eines radiographischen Abbildungsgerätes (100), in dem eine Strahlungsquelle (11), eine Vielzahl von Gittern (14, 15) und ein Strahlungsdetektor (16) nebeneinander in einer Strahlungsbestrahlungsachsenrichtung vorgesehen sind, veranlasst, zu funktionieren als:

ein Erzeuger (51), der ein rekonstruiertes Bild auf der Basis eines Moire-Streifenbildes erzeugt, das durch Ausführen eines Bildes durch Bestrahlung eines Subjekts mit Strahlung durch die Strahlungsquelle (11) erhalten wurde, wobei das Subjekt an einer Position angeordnet ist, die die Strahlungsbestrahlungsachsenrichtung überlappt;
ein Erfasser (51), der relative Positionsinformationen erfasst, die relative Positionen in zwei oder mehr Dimensionen des Subjekts in Bezug auf die Gitter (14, 15) sind; und
eine Steuerung (51), die das rekonstruierte Bild mit den relativen Positionsinformationen verknüpft.

## Revendications

1. Appareil d'imagerie radiographique (100) dans lequel une source de rayonnement (11), une pluralité de caillebotis (14, 15) et un détecteur de rayonnement (16) sont fournis côte à côte dans une direction d'axe d'irradiation de rayonnement, l'appareil d'imagerie radiographique comprenant :

un générateur (51) qui génère une image reconstruite sur une base d'une image de frange moirée obtenue en effectuant de l'imagerie par irradiation d'un sujet disposé à une position chevauchant la direction d'axe d'irradiation de rayonnement par la source de rayonnement (11) ;
un acquéreur (51) qui acquiert de l'information de position relative qui est des positions relatives en deux dimensions ou plus du sujet par rapport aux caillebotis (14, 15) ; et
un contrôleur (51) qui associe l'image reconstruite à l'information de position relative.

2. Appareil d'imagerie radiographique (100) selon la revendication 1, dans lequel l'acquéreur (51) acquiert l'information de position relative à partir d'une image capturée par un imageur (21) qui capture une image à partir de laquelle l'information de position relative peut être acquise.

3. Appareil d'imagerie radiographique (100) selon la revendication 1 ou 2,

dans lequel le générateur (51) génère une pluralité d'images reconstruites, et
le générateur (51) génère une image combinée en corrigeant des positions de la pluralité d'images reconstruites utilisant l'information de position relative.

4. Appareil d'imagerie radiographique (100) selon l'une quelconque des revendications 1 à 3,
dans lequel le générateur (51) combine les images reconstruites du sujet découpé dans un même échantillon parent en utilisant l'information de position relative du sujet découpé dans l'échantillon parent pour générer l'image reconstruite de l'échantillon parent.

5. Appareil d'imagerie radiographique (100) selon la revendication 1 ou 2,

dans lequel le générateur (51) génère une pluralité d'images reconstruites,
les images reconstruites sont des images de diffusion aux petits angles, et
le générateur (51) génère une image orientée en utilisant les images de diffusion aux petits angles à trois angles ou plus tournés autour de l'axe d'irradiation de rayonnement, pour lesquels les positions sont corrigées utilisant l'information de position relative.

6. Appareil d'imagerie radiographique (100) selon la revendication 1 ou 2,

dans lequel le générateur (51) génère une pluralité d'images reconstruites,
les images reconstruites sont des images de diffusion aux petits angles ou des images de phase différentielle, et
le générateur (51) génère une image en synthétisant les images de diffusion aux petits angles ou les images de phase différentielle à deux angles ou plus tournés autour de l'axe d'irradiation de rayonnement, pour lesquels des positions sont corrigées utilisant l'information de position relative.

7. Appareil d'imagerie radiographique (100) selon l'une quelconque des revendications 1 à 6,
dans lequel le générateur (51) associe l'information de position relative à l'information de coordonnée d'un modèle de conception assistée par ordinateur (CAD) pour générer des données dans un format qui peut être retransmis à l'ingénierie assistée par ordinateur (CAE).

8. Appareil d'imagerie radiographique (100) selon l'une quelconque des revendications 1 à 7,
dans lequel le contrôleur (51) détermine si de l'imagerie est possible ou non à partir d'information de matériau et/ou de composition du sujet et d'information d'épaisseur du sujet obtenues à partir de l'information de position relative.

9. Appareil d'imagerie radiographique (100) selon l'une quelconque des revendications 1 à 8,
dans lequel le contrôleur (51) déduit des conditions d'imagerie possibles à partir d'information de matériau et/ou de composition du sujet et d'information d'épaisseur du sujet obtenues à partir de l'information de position relative.

10. Méthode de génération d'image à être effectuée par un appareil d'imagerie radiographique (100) dans lequel une source de rayonnement (11), une pluralité de caillebotis (14, 15) et un détecteur de rayonnement (16) sont fournis côte à côte dans une direction d'axe d'irradiation de rayonnement, la méthode de génération d'image comprenant :

générer, par un générateur (51), une image reconstruite sur la base d'une image de frange moirée obtenue en effectuant de l'imagerie en irradiant un sujet disposé à une position chevauchant la direction d'axe d'irradiation de rayonnement avec du rayonnement par la source de rayonnement (11) ;
acquérir, par un acquéreur (51), de l'information de position relative qui est des positions relatives dans deux dimensions ou plus par rapport aux caillebotis (14, 15) ; et
associer, par un contrôleur (51), l'image reconstruite à l'information de position relative.

11. Système d'imagerie radiographique comprenant :

l'appareil d'imagerie radiographique (100) selon l'une quelconque des revendications 1 à 9 ; et
un appareil qui sort des données d'image à partir desquelles une plage d'imagerie et le sujet peuvent être saisis dans le même système de coordonnées,
dans lequel des positions d'un résultat d'analyse de l'appareil sont faites pour correspondre à des positions de l'image reconstruite utilisant l'information de position relative.

12. Système d'imagerie radiographique comprenant l'appareil d'imagerie radiographique (100) selon la revendication 2,

dans lequel des positions de l'image reconstruite et d'une image optique obtenue par l'imageur (21) sont alignées utilisant l'information de position relative, et l'image reconstruite et l'image optique sont affichées de manière superposée.

13. Programme entraînant un ordinateur d'un appareil d'imagerie radiographique (100) dans lequel une source de rayonnement (11), une pluralité de caillebotis (14, 15) et un détecteur de rayonnement (16) sont fournis côte à côte dans la direction d'axe d'irradiation de rayonnement, à fonctionner comme suit :

un générateur (51) qui génère une image reconstruite sur la base d'une image de frange moirée obtenue en effectuant de l'image en irradiant un sujet disposé à une position chevauchant la direction d'axe d'irradiation de rayonnement par la source de rayonnement (11) ;
un acquéreur (51) qui acquiert de l'information de position relative qui est des positions relatives dans deux dimensions ou plus du sujet par rapport aux caillebotis (14, 15) ; et
un contrôleur (51) qui associe l'image reconstruite à l'information de position relative.

# FIG.1

# FIG.2

PERIOD

12

z ⊗ → x

y

# FIG.3

5

51

CONTROLLER

52

OPERATOR

54

COMMUNICATION UNIT

53

DISPLAY

55

STORAGE

# FIG.4

# FIG.5

```
        ┌─────────────────┐
        │      START      │
        └─────────────────┘
                 │
    ┌────────────│
    │            ▼
    │   ┌─────────────────────────┐      S11
    │   │    CAPTURE Ref IMAGE     │
    │   └─────────────────────────┘
    │            │
    │            ▼
    │   ┌─────────────────────────┐      S12
    │   │    CAPTURE RGB-D IMAGE   │
    │   └─────────────────────────┘
    │            │
    │            ▼
    │   ┌─────────────────────────┐      S13
    │   │  ACQUIRE RELATIVE        │
    │   │  POSITION INFORMATION    │
    │   └─────────────────────────┘
    │            │
    │            ▼
    │   ┌─────────────────────────┐      S14
    │   │    CAPTURE Spl IMAGE     │
    │   └─────────────────────────┘
    │            │
    │            ▼              S15
    │          ╱───────────────╲
    │         ╱  IS POSITION     ╲
    └────────⟨ OF SUBJECT CHANGED? ⟩
      YES     ╲                 ╱
               ╲───────────────╱
                     │ NO
                     ▼               S16
        ┌─────────────────────────┐
        │  GENERATE RECONSTRUCTED  │
        │         IMAGE            │
        └─────────────────────────┘
                     │
                     ▼               S17
        ┌─────────────────────────┐
        │  DERIVE POSITION         │
        │  CORRECTION AMOUNT       │
        └─────────────────────────┘
                     │
                     ▼               S18
        ┌─────────────────────────┐
        │     COMBINE IMAGES       │
        └─────────────────────────┘
                     │
                     ▼
        ┌─────────────────┐
        │       END       │
        └─────────────────┘
```

# FIG .6

RGB-D CAMERA IMAGING RANGE — SUBJECT STAND

IMAGING
POSITION
A

SUBJECT

RGB-D CAMERA IMAGING RANGE — SUBJECT STAND

IMAGING
POSITION
B

SUBJECT

SUBJECT — SUBJECT STAND

IMAGING
POSITION
C

RGB-D CAMERA IMAGING RANGE

SUBJECT — SUBJECT STAND

IMAGING
POSITION
D

RGB-D CAMERA IMAGING RANGE

# FIG.7

SUBJECT STAND

RGB-D CAMERA IMAGING RANGE

IMAGING
POSITION

SUBJECT

SUBJECT STAND

RGB-D CAMERA
IMAGING
RANGE

IMAGING
POSITION

SUBJECT

SUBJECT STAND

RGB-D CAMERA
IMAGING
RANGE

IMAGING
POSITION

SUBJECT

## FIG.8

| SUBJECT STAND | |
|---|---|
| RGB-D CAMERA IMAGING RANGE | |
| | IMAGING POSITION |
| | SUBJECT |

⇨

| SUBJECT STAND | |
|---|---|
| RGB-D CAMERA IMAGING RANGE | |
| SUBJECT | IMAGING POSITION |

## FIG.9

PARENT SAMPLE

| CHILD SAMPLE 1 | CHILD SAMPLE 2 |
|---|---|
| CHILD SAMPLE 3 | CHILD SAMPLE 4 |
| CHILD SAMPLE 5 | CHILD SAMPLE 6 |

# FIG.10

START

INPUT MATERIAL/COMPOSITION INFORMATION — S21

CAPTURE RGB-D IMAGE — S22

ACQUIRE RELATIVE POSITION INFORMATION — S23

DETERMINE WHETHER OR NOT IMAGING IS POSSIBLE — S24

MAKE NOTIFICATION WHETHER OR NOT IMAGING IS POSSIBLE — S25

MAKE NOTIFICATION OF IMAGING POSSIBLE CONDITIONS — S26

END

# FIG.11

UPPER LEFT OF CAMERA IMAGING RANGE IS SET AT (x,y = 0,0)
SUBJECT STAND SURFACE IS SET AT z = 0

# FIG.12A

MODALITY(SEM)

RGB-D CAMERA

SUBJECT

SUBJECT STAND

SUBJECT STAND

RGB-D CAMERA IMAGING RANGE

SEM IMAGING RANGE

SUBJECT

# FIG.12B

TUBE

RGB-D CAMERA

G0 GRATING

SUBJECT

SUBJECT STAND

G1 GRATING

G2 GRATING

FPD

SUBJECT STAND

RGB-D CAMERA IMAGING RANGE

TALBOT IMAGING RANGE

SUBJECT

# FIG.13

A1

A2

DISPLAY EXAMPLE 1

RECONSTRUCTED IMAGE

SEM IMAGE

# FIG.14

DISPLAY EXAMPLE 2 — A3 / A4 / A5

| RECONSTRUCTED IMAGE | SEM IMAGE A | SEM IMAGE B |
| SEM IMAGE A | | |
| SEM IMAGE B | | |

# FIG.15

3D MODEL OF SUBJECT

↓

FLUID ANALYSIS RESULT (THREE-DIMENSIONAL INFORMATION)

↓

FLUID ANALYSIS RESULT (TWO-DIMENSIONAL INFORMATION) — A6

⟺

TUBE — RGB-D CAMERA
G0 GRATING
SUBJECT — SUBJECT STAND
G1 GRATING
G2 GRATING
FPD

↓

ORIENTED IMAGE — A7

**EP 4 197 445 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6780591 B
- US 2016042533 A1